(19) Europäisches Patentamt
European Patent Office
Office européen des brevets

(11) Veröffentlichungsnummer: **0 225 854**
**A1**

# (12) EUROPÄISCHE PATENTANMELDUNG

(21) Anmeldenummer: **86810574.3**

(22) Anmeldetag: **08.12.86**

(51) Int. Cl.⁴: **C 07 D 273/04**
A 01 N 43/88

(30) Priorität: **12.12.85 CH 5302/85**
**19.08.86 CH 3319/86**

(43) Veröffentlichungstag der Anmeldung:
**16.06.87 Patentblatt 87/25**

(84) Benannte Vertragsstaaten:
**AT BE CH DE ES FR GB IT LI NL**

(71) Anmelder: **CIBA-GEIGY AG**
**Klybeckstrasse 141**
**CH-4002 Basel (CH)**

(72) Erfinder: **Farooq, Saleem, Dr.**
**Kirchackerstrasse 27**
**CH-4422 Arisdorf (CH)**

**Gallay, Jean Jacques, Dr.**
**Blumenrain 19**
**CH-4312 Magden (CH)**

Die Bezeichnung der Erfindung wurde geändert (Richtlinien für die Prüfung im EPA, A-III, 7.3).

(54) **Substituierte Oxadiazinone als Schädlingsbekämpfungsmittel.**

(57) Schädlingsbekämpfungsmittel enthaltend als Wirkstoff ein Oxadiazin-2-on der Formel I

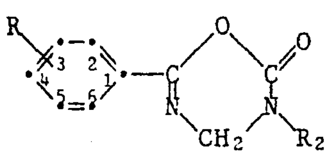

(I),

worin
R $S(O)_n-R_1$;
$R_1$ $C_1-C_6$-Alkyl;
$R_2$ $C_1-C_6$-Alkyl oder $C_3-C_6$-Cycloalkyl;
n eine Zahl 0, 1 oder 2 bedeuten.

Es werden insektizide und akarizide Mittel enthaltend einen Wirkstoff der Formel I, Mischungen insektizider und akarizider Wirkstoffe, sowie neuartige, unter Formel I fallende Oxadiazin-2-one und Verfahren zu ihrer Herstellung beschrieben.

EP 0 225 854 A1

**Beschreibung**

Schädlingsbekämpfungsmittel

Die vorliegende Erfindung betrifft 1,3,5-Oxidiazin-2-one, Verfahren zu ihrer Herstellung und ihre Verwendung als Insektizide und Akarizide.

Die erfindungsgemäss verwendeten und in den erfindungsgemässen Mitteln als Wirkstoffe enthaltenen Oxadiazin-2-one haben die Formel I

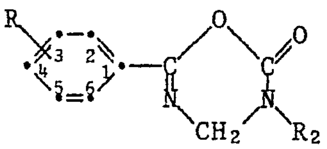

(I),

worin
R $S(O)_n$-$R_1$;
$R_1$ $C_1$-$C_6$-Alkyl;
$R_2$ $C_1$-$C_6$-Alkyl oder $C_3$-$C_6$-Cycloalkyl und
n eine Zahl 0, 1 oder 2 bedeuten.

Die Alkylgruppen bei $R_1$ und $R_2$ können geradkettig oder verzweigt sein und haben in der Kette 1 bis 6 Kohlenstoffatome. Beispiele dieser Gruppen sind:
Methyl, Aethyl, n-Propyl, Isopropyl, n-Butyl, n-Pentyl und n-Hexyl sowie deren Isomere. Die Cycloalkylgruppen bei $R_2$ sind: Cyclopropyl, Cyclobutyl, Cyclopentyl und Cyclohexyl.

Die Erfindung betrifft daneben auch neuartige Verbindungen der Formel I,

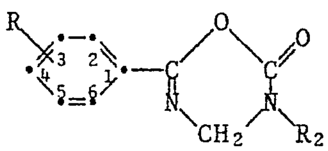

(I),

worin
R $S(O)_n$-$R_1$;
$R_1$ $C_1$-$C_6$-Alkyl;
$R_2$ $C_1$-$C_6$-Alkyl oder $C_3$-$C_6$-Cycloalkyl und
n eine Zahl 0 oder 1 bedeuten,
mit der Massgabe, dass $R_2$ eine von Methyl abweichende Bedeutung hat, wenn R für 4-$SOCH_3$ steht.

Hervorzuheben sind neuartige Verbindungen der Formel I,
worin
$R_1$ $C_1$-$C_4$-Alkyl und
$R_2$ $C_1$-$C_4$-Alkyl, Cyclopropyl oder Cyclohexyl
bedeuten.

Besonders wertvoll sind die neuartigen Verbindungen der Formel I worin n = 0 ist sowie diejenigen gemäss dem nachfolgenden Beispiel 1.

Besonders hervorzuheben sind die neuartigen Verbindungen der Formel I,
worin
$R_1$ Methyl und
$R_2$ Isopropyl;
bedeuten.

Als Beispiele für Verbindungen der Formel I seien die folgenden genannt:

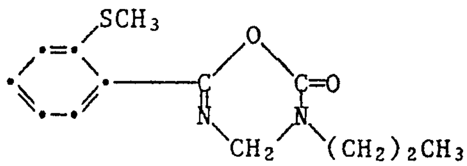

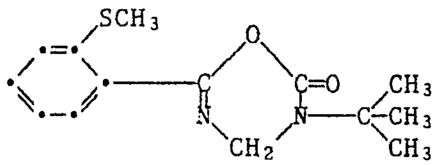

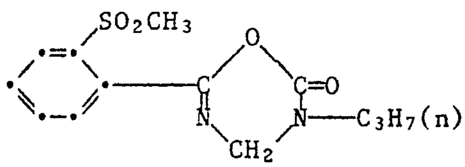

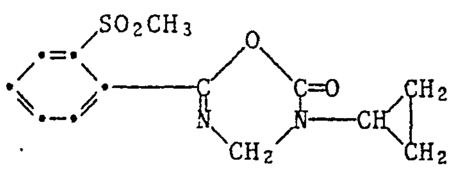

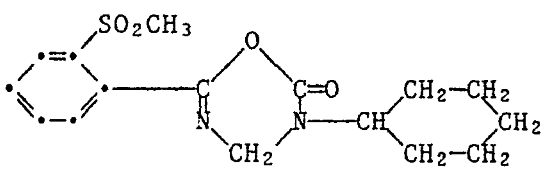

Die Verbindungen der Formel I werden in an sich bekannter Weise hergestellt, z.B. indem man eine Verbindung der Formel II

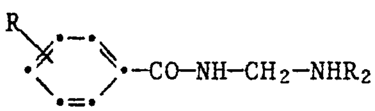 (II),

worin R, $R_1$, $R_2$ und n die für Formel I angegebenen Bedeutungen haben, mit Phosgen cyclisiert. Dieses Verfahren kann insofern modifiziert werden, als man eine Verbindung der Formel II durch Umsetzung einer Verbindung der Formel III

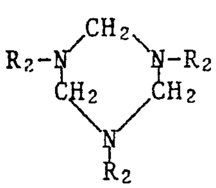 (III)

mit einem Benzamid der Formel IV

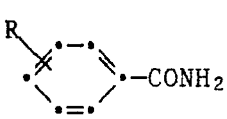 (IV),

in situ herstellt und dann direkt mit Phosgen cyclisiert, wobei in den Formeln II bis IV R, $R_1$, $R_2$ und n die für die Formel I angegebene Bedeutung haben. Statt Phosgen können auch Kohlensäuredialkylester oder

Halogenameisensäureester, besonders Chlorameisensäureester, zur Cyclisierung dienen; insbesondere sind für diesen Zweck Alkylester mit $C_1$-$C_4$-Alkyl geeignet.

Die Cyclisierung einer Verbindung der Formel II wird bei einer Temperatur zwischen -50° und +120° gegebenenfalls in Gegenwart einer Base und in gegenüber den Reaktionsteilnehmern inerten Lösungs- und/oder Verdünnungsmitteln durchgeführt.

Als Lösungs- oder Verdünnungsmittel kommen dabei aliphatische oder aromatische Kohlenwasserstoffe wie Benzol, Toluol, Xylole, Hexan; Halogenkohlenwasserstoffe wie Chloroform, Methylenchlorid; Ketone, wie Aceton, Methyläthylketon; Nitrile wie Acetonitril; Dimethylformamid oder Dimethylsulfon; insbesondere jedoch Aether und ätherartige Verbindungen, wie Dialkyläther, Dioxan, Tetrahydrofuran, 1,2-Dimethoxyäthan sowie zweiphasige Gemische, z.B. wie Wasser/Benzol in Betracht. Als Basen sind insbesondere tertiäre Amine, z.B. Trialkylamine, Pyridin oder Pyridinbasen, aber auch NaH oder im Falle wasserhaltiger Gemische Alkali- oder Erdalkalihydroxide oder -carbonate zu nennen.

Wie vorstehend angegeben lassen sich die Verbindungen der Formel I vorteilhafterweise auch im Eintopfverfahren (in situ) direkt aus Benzamiden der Formel IV herstellen, indem man letztere in Gegenwart einer wasserfreien Säure, vorzugsweise einer Halogenwasserstoffsäure, mit einem Triazin der Formel III in einem inerten organischen Lösungsmittel umsetzt und anschliessend - ohne das Zwischenprodukt der Formel II zu isolieren - mit Phosgen, vorzugsweise in Gegenwart einer Base, die Cyclisierung durchführt. Diese spezielle Ausführungsform des Verfahrens zur Herstellung der Verbindungen der Formel I bietet den Vorteil, dass auch solche Verbindungen der Formel II, die säureempfindlich sind, ohne Schwierigkeiten cyclisiert werden können.

Die Ausgangsstoffe der vorstehenden Formel II, III und IV sind bekannt oder lassen sich analog bekannten Verfahren in üblicher Weise herstellen.

Es ist bereits aus der DE-OS 2 459 413 bekannt, 6-Phenyl-1,3,5-oxadiazin-2-one, die in 3-Stellung gegebenenfalls durch Alkyl, Alkoxyalkyl, Alkenyl oder Cycloalkyl substituiert sind, als Herbizide und Coccidiostatika zu verwenden. Aehnliche Verbindungen werden in der CH-PS 630 245 als Beifuttermittel vorgeschlagen. Ferner werden in 6-Stellung durch einen heterozyklischen Rest substituierte 1,3,5-Oxadiazin-2-one und ihre Verwendung zur Bekämpfung von Coccidien in der DE-OS 2 624 341 beschrieben.

Demgegenüber wurde überraschenderweise gefunden, dass sich Verbindungen der Formel I bei guter Pflanzenverträglichkeit und geringer Warmblütertoxizität zur Bekämpfung von verschiedenartigen Insekten und Vertretern der Ordnung Acarina an Tieren und Pflanzen sowie im Boden eignen, insbesondere auch als ausgezeichnete Synergisten für Verbindungen anderer Wirkstoffklassen.

Die Erfindung betrifft somit auch insektizide und akarizide Mittel enthaltend als Wirkstoff eine Verbindung der Formel I,

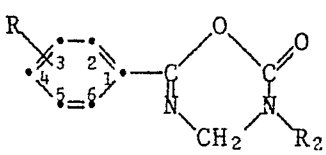

(I),

worin
R $S(O)_n$-$R_1$;
$R_1$ $C_1$-$C_6$-Alkyl;
$R_2$ $C_1$-$C_6$-Alkyl oder $C_3$-$C_6$-Cycloalkyl und
n eine Zahl 0, 1 oder 2 bedeuten, zusammen mit geeigneten Hilfs-und/oder Trägerstoffen.

Bevorzugt werden erfindungsgemäss insektizide und akarizide Mittel enthaltend als Wirkstoff eine Verbindung der Formel I,
worin
R $SR_1$;
$R_1$ $C_1$-$C_4$-Alkyl und
$R_2$ $C_1$-$C_4$-Alkyl, Cyclopropyl oder Cyclohexyl;
bedeuten.

So können die Verbindungen der Formel I zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Acarina eingesetzt werden.

Die Verbindungen der Formel I sind einsetzbar zur Bekämpfung von pflanzenschädigenden Insekten in Zier-und Nutzpflanzen, insbesondere in Baumwoll- und Reiskulturen (z.B. Spodoptera littoralis, Heliothis virescens, Nephotettix cincticeps, Chilo suppressalis und Laodelphax striatellus) sowie Gemüse- und Obstkulturen (z.B. Leptinotarsa decemlineata, Myzus persicae, Laspeyresia pomonella und Adoxophyes reticulana) und von Bodeninsekten (z.B. Aulacophora femoralis, Chortophila brassicae, Diabrotica balteata, Pachnoda savignyi und Scotia ypsilon). Besonders hervorzuheben ist die hohe systemische Wirksamkeit, die vor allem bei der Bekämpfung saugender Insekten und phytopathogener Pilze zum Tragen kommt.

Wirkstoffe der Formel I zeigen auch eine Wirkung gegen Fliegen,wie z.B. Musca domestica und

Mückenlarven. Ausserdem besitzen die Verbindungen der Formel I auch nematizide Eigenschaften, ferner zeichnen sie sich durch eine breite, ovizide und ovolarvizide Wirkung aus und besitzen eine wertvolle Wirkung gegen ektoparasitäre Milben und Zecken, z.B. der Familien Ixodidae, Argasidae und Dermanyssidae.

Die Verbindungen der Formel I wirken auch gegen phytopathogene Pilze. So wirken Verbindungen der Formel I gegen die den folgenden Klassen angehörenden phytopathogenen Pilze: Ascomycetes (z.B. Erysiphaceae, Fusarium, Helminthosporium); Basidiomycetes wie Puccinia, Rhizoctonia, Tilletia, Hemileia; Fungi imperfecti (z.B. Cercospora, Botrytis, Septoria); Phycomycetes wie Phytophthora.

Die Verbindungen der Formel I können zur Behandlung von Saatgut und Vorrat (Früchte, Knollen, Körner) und Pflanzenstecklingen oder zur Saatfurchenapplikation zum Schutz vor Pilzinfektionen, Insekten und Vertretern der Ordnung Akarina, sowie gegen im Erdboden auftretende phytopathogene Pilze und Insekten eingesetzt werden.

Zusätzlich wurde nun gefunden, dass Kombinationen von Verbindungen der Formel I mit anderen Insektiziden und/oder Akariziden, wie z.B. org. Phosphorverbindungen; Imidoäthern; Amidinen; Aminen; Hydrazinen; Triazinen; Harnstoffen; pyrethrinartige Verbindungen sowie Diphenylmethanderivaten und Karbamaten, in einem Gew.-Verhältnis von 20:1 bis 1:20, vorzugsweise 5:1 bis 1:5, eine potenzierende Wirkung gegen verschiedenartige Schädlinge an Tieren und Pflanzen entfalten, welche die additive Wirkung dieser kombinierten Wirkstoffe überraschenderweise stark übertrifft. Diese potenzierende Wirkung der kombinierten Wirkstoffe tritt bei allen Bekämpfungsverfahren, besonders bei der Bekämpfung saugender Schadinsek ten, auf. So können erfindungsgemäss Verfahren eingesetzt werden, wobei die Wirkstoffe vorgemischt aus einem Behälter oder zeitlich kurz nacheinander oder simultan aus verschiedenen Behältern in den Lebensraum der Schädlinge appliziert werden.

Einen potenzierenden Effekt erzeugen Verbindungen der Formel I insbesondere in Mischungen mit z.B. folgenden, bekannten Insektiziden oder Akariziden:

1. Org. Phosphorester.

1.1 Alkyl-(Acyl-)Phosphate:

Acephat; Methamidophos; Ethoprophos; Disulfoton; TEPP; Naled; Carbophenothion; Trichlorphon; Ethion; Sulfotep; Mipafox; Vamidothion; Phenkapton; Terbufos; Chlormephos; Phoxim; Phorat und Fospargyl.

1.2 Carbonylalkylphosphate:

Phenthoat; Dimethoat; Malathion; Morphotion und Formothion.

1.3 Vinylphosphate.

Tetrachlorvinphos; Propetamphos; Methacrifos; Chlorfenvinphos; Tetrachlorvinphos-äthyl; Thiophosdrin®; Mevinphos; Crotoxyphos; Phosphamidon; Dicrotophos; Monocrotophos; Dichlorvos; Fosfinon®; Akton®; Bomyl®; Bromfenvinphos und Heptenophos.

1.4 Aromatische Phosphate:

Profenofos; Trifenophos; Bromophos; Mercaptoprophos; Methylparathion; Parathion; Chlorthion; Fenitrothion; Fenchlorphos; Fenthion; Cyanophos; Dichlofenthion; Fenamiphos; Fonofos; Jodfenfos; Temephos; Prothiophos; Isofenphos; Fensulfothion und Lepthophos.

1.5 Heterocyclische Phosphate:

Isazophos; Triazophos; Chlorpyrifos; Phosmet; Azamethiphos; Phosalon; Azinphos-methyl; Azinphos-äthyl; Dialifos; Dioxathion; Diazinon; Methidathion; Isozathion; Chlorpyrofos-methyl; Phosfolan; Fosthietan; Etrimfos; Pyridafenthion; Mephosfolan; Thionazin; Zolaprofos und Purimiphos-methyl.

2. Carbamate:

2.1 Aromatische Carbamate:

Dioxacarb; Methiocarb; Isoprocarb; Carbaryl; Xylylcarb; CPMC, Bufencarb; BPMC; Carbofuran; Propoxur, Ethiofencarb; Carbosulfan; Mydrol®; Bendiocarb; Aminocarb; Chloetocarb und Benzofuracarb.

2.2 Heterocyclische Carbamate:

Isolan®; Dimetilan; Primicarb; Hyquincarb; Methomyl; Aldicarb; Tirpat®; Demethyl-oxamyl; Thiodicarb; Nitrilacarb; Oxamyl; Thiofanox und Butocarboxim.

3. Diphenylmethanderivate:

DDT; Methoxychlor; Prolan; Bulan; Chlorbenzilat; Chlorpropylat und Brompropylat.

4. Amidine, Imidoäther, Amine, Hydrazine:

Chlordimeform; Benzomat und Amitraz.

5. Pyrethrinartige Verbindungen:

Resmethrin; Permethrin; Phenothrin; Cypermethrin; Decamethrin; Fenpropathrin; Fenfluthrin; Fenpyrithrin; Cyhalothrin; Flumethrin; Cyfluthrin; Fenvalerat; Fluvalinat und Flucytrin.

6. Harnstoff und Triazine.

Diflubenzuron; Flucarbenzuron und Cyromazin.

Die Verbindungen der Formel I oder deren Kombinationen mit anderen Wirkstoffen werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln,

Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I oder dessen Kombination mit anderen Wirkstoffen und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Essigester, Propylmyristat oder Propylpalmitat, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; Silikonöle oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I oder dessen Kombination mit andern aktiven Verbindungen nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls subsituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind auch die Fettsäuremethyl-taurinsalze zu erwähnen.

Häufiger werden jedoch sog. synthetische Tenside verwendet, insbesondere Fettsulfonate, sulfonierte Benzimidazolderivate oder Alkylarlsulfonate.

Die Fettsulfonate oder -sulfate liegen in der Regel als Alkali-, Erdalkali- oder gegebenenfalls substituierte Ammoniumsalze vor und weisen einen alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefeläureester und Sulfonsäure von Fettalkohol-Aethylenoxid-Addukten. Die sulfonierten Benzimidazolderivaten enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze oder Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigte oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionisher Tenside seien Nonylphenolpolyäthoxyäthanole, Ricinusölpolyglycoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartinäre Ammoniumsalze, welche als N-Substituentenmindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben: "McCutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 Gew.%, insbesondere 0,1 bis 95 Gew.%, Wirkstoff der Formel I, 1 bis 99,9 Gew.% eines festen oder flüssigen Zusatzstoffes und 0 bis 25 Gew.%, insbesondere 0,1 bis 25 Gew.%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1

Herstellung von 6-(2-Methylthiophenyl)-3-isopropyl-3,4-dihydro-2H-1,3,5-oxadiazin-2-on:

In 220 ml Dimethoxyäthan werden bei 20°C 5,5 g gasförmiger Chlorwasserstoff eingeleitet. Zum auf -30°C abgekühlten Reaktionsgemisch werden 6,4 g 1,3,5-Tri-isopropyl-2,4,6-hexahydrotriazin gelöst in 30 ml Dimethoxyäthan zugetropft und anschliessend 15 g 2-Methylthiobenzamid zugesetzt. Nach zweistündigem Rühren bei 20°C werden wiederum bei -30°C 56 ml einer 20 %igen Phosgenlösung in Toluol und anschliessend eine Lösung von 13 ml Pyridin in 30 ml Dimethoxyäthan zugetropft. Nach einstündigem Rühren bei 0 - 5°C werden noch 16 ml Pyridin tropfenweise zugesetzt. Das Gemisch wird dann während 2 Stunden bei 60°C gerührt, abgekühlt, filtriert und eingedampft.

Der Rückstand wird in Essigester aufgenommen, zweimal mit Wasser und zweimal mit einer gesättigten Kochsalzlösung ausgeschüttelt. Die abgetrennte organische Phase wird über Natriumsulfat getrocknet und eingedampft. Das Rohprodukt wird aus Hexan/Essigester (1:1) umkristallisiert.

Man erhält so die Titelverbindung Nr. 1.1 der Formel

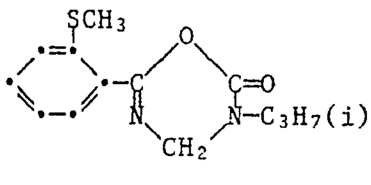

mit einem Schmelzpunkt von 53 - 54°C.

In analoger Weise werden auch folgende Verbindungen hergestellt:

mit einem Schmelzpunkt von 53 - 54°C.

In analoger Weise werden auch folgende Verbindungen hergestellt:

Nr. 1.2

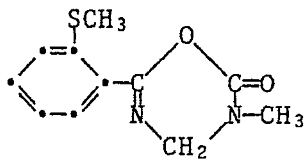

Smp.: 93-95°C

Nr. 1.3

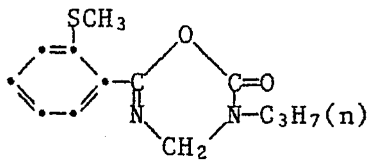

Smp.: 65-66°C

Nr. 1.4

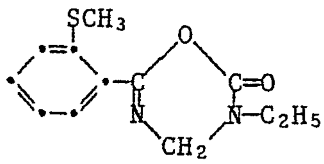

Smp.: 66-69°C

Nr. 1.5

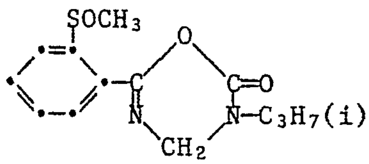

Smp.: 134-135°C

Nr. 1.6

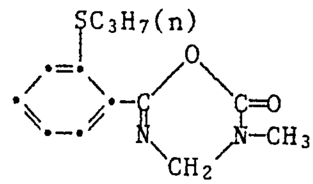

Smp.: 55-56°C

Nr. 1.7

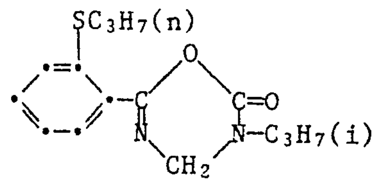

$n_D^{20°}$ = 1,5766

Nr. 1.8

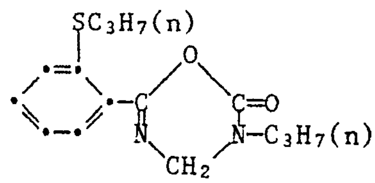

$n_D^{20°}$ = 1,5750

Nr.1.9

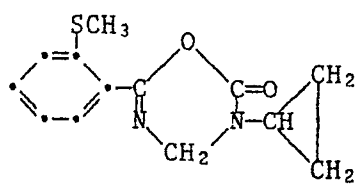

Smp.:103-105°C

Nr. 1.10

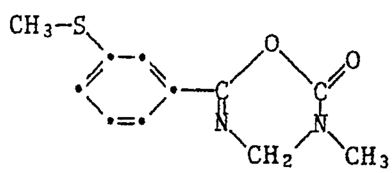

Smp.: 120-121°C

Nr. 1.11

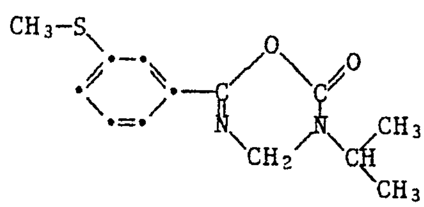

Smp.: 41-43°C

Nr. 1.12

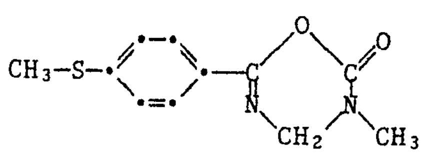

Smp.: 150-151°C

Nr. 1.13

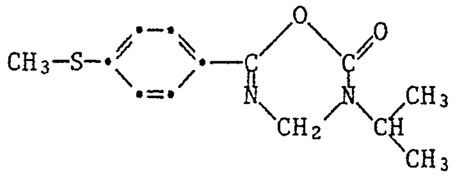

Smp.: 80-81°C

Nr. 1.14

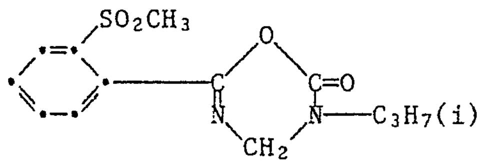

Smp. 177 - 182°C

Wie vorstehend angegeben sind auch folgende Verbindungen der Formel I herstellbar:

| R | $R_2$ |
|---|---|
| $2-SO_2-CH_3$ | $C_3H_7(i)$ |
| $2-S-CH_3$ | $C_3H_7(n)$ |
| $2-S-CH_3$ | $C_4H_9(t)$ |
| $2-SO_2-CH_3$ | $C_3H_7(n)$ |
| $2-SO_2-CH_3$ | 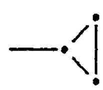 |
| $2-SO_2-CH_3$ | 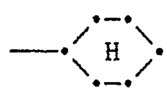 |
| $2-S-C_4H_9(n)$ | $CH_3$ |
| $2-SO-C_4H_9(n)$ | $CH_3$ |
| $2-S-C_4H_9(n)$ | $C_3H_7(i)$ |
| $2-SO-C_4H_9(n)$ | $C_3H_7(i)$ |
| $2-S-C_3H_7(n)$ | $CH_3$ |
| $2-SO-C_3H_7(n)$ | $CH_3$ |
| $2-S-C_3H_7(n)$ | $C_3H_7(i)$ |
| $2-SO-C_3H_7(n)$ | $C_3H_7(i)$ |
| $2-S-C_5H_9(n)$ | $CH_3$ |
| $2-SO-C_5H_9(n)$ | $CH_3$ |
| $2-S-C_5H_9(n)$ | $C_3H_7(i)$ |
| $2-SO-C_5H_9(n)$ | $C_3H_7(i)$ |
| $2-S-C_4H_9(t)$ | $CH_3$ |
| $2-SO-C_4H_9(t)$ | $CH_3$ |
| $2-S-C_4H_9(t)$ | $C_3H_7(i)$ |
| $2-SO-C_4H_9(t)$ | $C_3H_7(i)$ |
| $2-S-C_4H_9(s)$ | $CH_3$ |
| $2-SO-C_4H_9(s)$ | $CH_3$ |
| $2-S-C_4H_9(s)$ | $C_3H_7(i)$ |

| R | $R_2$ |
|---|---|
| 2-SO-$C_4H_9$(s) | $C_3H_7$(i) |
| 2-S-$C_4H_9$(i) | $CH_3$ |
| 2-SO-$C_4H_9$(i) | $CH_3$ |
| 2-S-$C_4H_9$(i) | $C_3H_7$(i) |
| 2-SO-$C_4H_9$(i) | $C_3H_7$(i) |
| 2-S-$C_3H_7$(i) | $CH_3$ |
| 2-SO-$C_3H_7$(i) | $CH_3$ |
| 2-S-$C_3H_7$(i) | $C_3H_7$(i) |
| 2-SO-$C_3H_7$(i) | $C_3H_7$(i) |
| 4-S-$C_4H_9$(n) | $CH_3$ |
| 4-SO-$C_4H_9$(n) | $CH_3$ |
| 4-S-$C_4H_9$(n) | $C_3H_7$(i) |
| 4-SO-$C_4H_9$(n) | $C_3H_7$(i) |
| 4-S-$C_3H_7$(n) | $CH_3$ |
| 4-SO-$C_3H_7$(n) | $CH_3$ |
| 4-S-$C_3H_7$(n) | $C_3H_7$(i) |
| 4-SO-$C_3H_7$(n) | $C_3H_7$(i) |
| 4-S-$C_5H_{11}$(n) | $CH_3$ |
| 4-SO-$C_5H_{11}$(n) | $CH_3$ |
| 4-S-$C_5H_{11}$(n) | $C_3H_7$(i) |
| 4-SO-$C_5H_{11}$(n) | $C_3H_7$(i) |
| 4-S-$C_4H_9$(i) | $CH_3$ |
| 4-SO-$C_4H_9$(i) | $CH_3$ |
| 4-S-$C_4H_9$(i) | $C_3H_7$(i) |
| 4-SO-$C_4H_9$(i) | $C_3H_7$(i) |
| 4-S-$C_4H_9$(s) | $CH_3$ |
| 4-SO-$C_4H_9$(s) | $CH_3$ |
| 4-S-$C_4H_9$(s) | $C_3H_7$(i) |
| 4-SO-$C_4H_9$(s) | $C_3H_7$(i) |
| 4-S-$C_3H_7$(i) | $CH_3$ |
| 4-SO-$C_3H_7$(i) | $CH_3$ |
| 4-S-$C_3H_7$(i) | $C_3H_7$(i) |
| 4-SO-$C_3H_7$(i) | $C_3H_7$(i) |

Beispiel 2: Formulierungsbeispiele für Wirkstoffe gemäss dem Herstellungsbeispiel 1 oder deren Kombinationen mit andern Wirkstoffen

(% = Gewichtsprozent)

| 2.1 Emulsions-Konzentrate | a) | b) | c) | d) |
|---|---|---|---|---|
| Wirkstoff gemäss dem Herstellungsbeispiel 1 oder dessen Kombination mit andern Wirkstoffen | 10 % | 25 % | 40 % | 50 % |
| Ca-Dodecylbenzolsulfonat | - | 5 % | 8 % | 6 % |

| | | | | |
|---|---|---|---|---|
| Ricinusöl-polyäthylenglykoläther (36 Mol AeO) | - | 5 % | - | - |
| Tributylphenol-polyäthylenglykoläther (30 Mol AeO) | - | - | 12 % | 4 % |
| Ricinusölthioxilat | 25 % | - | - | - |
| Cyclohexanon | - | - | 15 % | 20 % |
| Butanol | 15 % | - | - | - |
| Xylolgemisch | - | 65 % | 25 % | 20 % |
| Essigester | 50 % | - | - | - |

Aus solchen Konzentraten können durch Verdünnen mit Wasser Emulsionen jeder gewünschten Konzentration hergestellt werden.

| 2.2 Lösungen | a) | b) |
|---|---|---|
| Wirkstoff gemäss dem Herstellungsbeispiel 1 oder dessen Kombination mit andern Wirkstoffen | 10 % | 5 % |
| Aethylenglykol-monomethyläther | - | - |
| Polyäthylenglykol (MG 400) | 70 % | - |
| N-Methyl-2-pyrrolidon | 20 % | - |
| Epoxidiertes Kokosnussöl | - | 1 % |
| Benzin (Siedegrenzen 160 - 190°C) | - | 94 % |

Die Lösungen sind zur Anwendung in Form kleinster Tropfen geeignet.

| 2.3 Granulate | a) | b) |
|---|---|---|
| Wirkstoff gemäss dem Herstellungsbeispiel 1 oder dessen Kombination mit andern Wirkstoffen | 5 % | 10 % |
| Kaolin | 94 % | - |
| Hochdisperse Kieselsäure | 1 % | - |
| Attapulgit | - | 90 % |

Der Wirkstoff oder dessen Kombination mit andern Wirkstoffen wird in Methylenchlorid gelöst, auf den Träger aufgesprüht und das Lösungsmittel anschliessend im Vakuum angedampft.

2.4 Stäubemittel

| | a) | b) | c) | d) |
|---|---|---|---|---|
| Wirkstoff gemäss dem Herstellungsbeispiel 1 oder dessen Kombination mit andern Wirkstoffen | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff erhält man gebrauchsfertige Stäubemittel.

2.5 Spritzpulver

| | a) | b) | c) |
|---|---|---|---|
| Wirkstoff gemäss dem Herstellungsbeispiel 1 oder dessen Kombination mit andern Wirkstoffen | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 67 % | 27 % | - |

Der Wirkstoff oder dessen Kombination mit andern Wirkstoffen wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6 Extruder Granulat

Wirkstoff gemäss dem
Herstellungsbeispiel 1 oder dessen Kombination
mit andern Wirkstoffen 10 %
Na-Ligninsulfonat 2 %
Carboxymethylcellulose 1 %
Kaolin 87 %

Der Wirkstoff oder dessen Kombination mit andern Wirkstoffen wird mit den Zusatzstoffen vermischt, vermahlen und mit Wasser angefeuchtet. Dieses Gemisch wird extrudiert und anschliessend im Luftstrom getrocknet.

2.7 Umhüllungs-Granulat

Wirkstoff gemäss dem
Herstellungsbeispiel 1 oder dessen Kombination
mit andern Wirkstoffen 3 %
Polyäthylenglykol (MG 200) 3 %
Kaolin 94 %

Der feingemahlene Wirkstoff oder dessen Kombination mit andern Wirkstoffen wird in einem Mischer auf das mit Polyäthylenglykol angefeuchtete Kaolin gleichmässig aufgetragen. Auf diese Weise erhält man staubfreie Umhüllungs-Granulate.

2.8 Suspensions-Konzentrat

Wirkstoff gemäss dem Herstellungsbeispiel 1 oder dessen Kombination mit anderen Wirkstoffen 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff oder dessen Kombination mit anderen Wirkstoffen wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologiebeispiele für Wirkstoffe gemäss dem Herstellungsbeispiel 1

3.1. Wirkung gegen Lucilia sericata

Zu 9 ml eines Zuchtmediums wird bei 50°C ein ml einer 0,1 % Aktivsubstanz enthaltenden wässrigen Zubereitung hinzugefüht. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Beispiel 1 zeigen in diesem Test ein 80 - 100%ige Wirkung gegen Lucilia sericata.

3.2.Wirkung gegen Aedes aegypti

Auf die Oberfläche von 150 ml Wasser, das sich in einem Behälter befindet, wird so viel einer 0,1%igen acetonischen Lösung des Wirkstoffs pipettiert, dass eine Konzentration von 12,5 ppm erhalten wird. Nach Verdunsten des Acetons wird der Behälter mit 30 bis 40 2tägigen Aëdes-Larven beschickt. Nach 2 und 7 Tagen wird die % Mortalität (Anzahl der schwimmunfähigen Larven) geprüft.

Verbindungen gemäss Beispiel 1 zeigen 100%ige Wirkung (Mortalität) im obigen Test.

3.3. Ovizide Wirkung auf Heliothis virescens

Entsprechende Mengenanteile einer benetzbaren pulverförmigen Formulierung, enthaltend 25 Gew.% des zu prüfenden Wirkstoffes, werden mit jeweils so viel Wasser vermischt, dass sich wässrige Emulsionen mit Wirkstoffkonzentrationen von 400 bis 12,5 ppm ergeben.

In diese wirkstoffhaltigen Emulsionen werden eintägige Eigelege von Heliothis auf Cellophan® während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgelegt. Zur Auswertung wird die zur 100%igen Abtötung der Eier erforderliche Wirkstoffkonzentration bestimmt.

Verbindungen gemäss Beipiel 1 zeigt bei 12,5 ppm 100%ige Wirkung (Mortalität) im obigem Test.

3.4. Wirkung gegen Bodeninsekten (Diabrotica balteata)

Es werden 5 etwa einen bis drei cm lange Maiskeimlinge sowie eine Rondelle aus Filterpapier in eine 400 ppm des zu prüfenden Wirkstoffes enthaltende wässrige Zubereitung getaucht. Die feuchte Filterpapierrondelle wird auf dem Boden eines 200 ml Plastik-bechers ausgelegt, und dann werden die 5 behandelten Maiskeimlinge zusammen mit 10 Larven von Diabrotica balteata des zweiten bis dritten Larvalstadiums in den Becher gegeben. Pro Wirkstoffkonzentration werden 2 Ansätze durchgeführt. Die mit den Larven besetzten Becher werden während 6 Tagen bei Tageslicht, einer relativen Luftfeuchtigkeit von 40 bis 60% und einer Temperatur von 22 bis 24°C gehalten. Danach wird die prozentuale Abtötung der Testtiere bestimmt.

Verbindungen gemäss Beispiel 1 zeigen in diesem Test gute Wirkung.

3.5. Wirkung gegen Zecken

A) Amblyomma hebraeum

50 Nymphen werden in ein Glasröhrchen gezählt und für 1 bis 2 Minuten in 2 ml einer wässrigen Emulsion mit 10 ppm Testsubstanz getaucht. Das Röhrchen wird dann mit einem genormten Wattebausch verschlossen und auf den Kopf gestellt, damit die Wirkstoffemulsion von der Watte aufgenommen werden kann.

Die Auswertung erfolgt nach 1 Woche. Für jeden Versuch werden 2 Wiederholungen durchgeführt.

B) Boophilus microplus (Larven)

Mit einer analogen Verdünnungsreihe wie beim Test A) werden mit je 20 sensiblen resp. OP-resistenten Larven Versuche durchgeführt. (Die Resistenz bezieht sich auf die Verträglichkeit von Diazinon).

Die Verbindungen gemäss dem Herstellungsbeispiel 1 zeigen eine gute Wirkung gegen Nymphen bzw.

Larven der Zecken: Amblyomma hebraeum und Boophilus microplus.

3.6 Wirkung gegen Laodelphax striatellus und Nilaparvata lugens (Nymphen)

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit jeweils 100 ml einer acetonischen Lösung enthaltend 800 ppm Wirkstoff besprüht. Nach dem Antrocknen des Spritzbelages erfolgt die Besiedlung jeder Pflanze mit je 20 Nymphen der Testtiere im dritten Stadium. Um die Zikaden am entweichen zu hindern, wird über die besiedelten Pflanzen jeweils ein Glaszylinder gestülpt und dieser mit einem Gaze-Deckel abgedeckt. Die Nymphen werden bis zum Erreichen des folgenden Ent wicklungsstadiums über 10 Tage an der behandelten Pflanze gehalten. Die Auswertung auf %-Mortalität erfolgt 1, 4 und 8 Tage nach der Behandlung.

Erfindungsgemässe Verbindungen gemäss Beispiel 1 zeigen gute Wirksamkeit in diesem Test.

3.7 Wirkung gegen Laodelphax striatellus und Nilaparvata lugens (ovizid)

Der Test wird an wachsenden Pflanzen durchgeführt. Jeweils 4 Reispflanzen (Dicke des Stengels 8 mm, Höhe ca. 20 cm) werden in Töpfe (Durchmesser von 8 cm) eingepflanzt.

Die Pflanzen werden auf einem Drehteller mit jeweils 100 ml einer acetonischen Lösung enthaltend 400 ppm Wirkstoff besprüht. Nach dem Weibchen besiedelt. Um die Tiere am Entweichen zu hindern, wird über jede besiedelte Pflanze ein Glaszylinder gestülpt und dieser mit einem Gaze-abgedeckt. Die Weibchen bleiben 4 Tage zur Eiablage auf der behandelten Pflanze und werden dann entfernt.

Etwa 8 Tage nach Besiedlung schlüpfen die jungen Zikaden, und es erfolgt deren Auszählung. Aus dem Vergleich der Anzahl geschlüpfter Larven auf den behandelten Pflanzen zu den auf den unbehandelten Kontrollpflanzen geschlüpften Tieren wird die prozentuale Mortalität berechnet.

Verbindungen gemäss Beispiel 1 zeigen in obigem Test gute ovizide Wirkung.

3.8 Potenzierung der insektiziden Kontaktwirkung: Myzus persicae (OP-resistent)

3.8.1.

A. Verbindung Nr. 1.1 gemäss Herstellungsbeispiel 1 der Formel

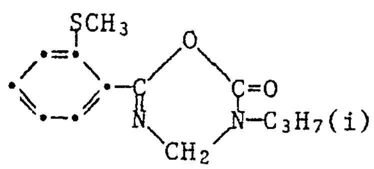

als 20%iges Spritzpulver

B. Phosphamidon der Formel

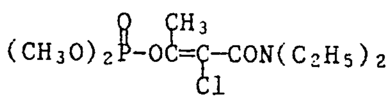

als 20%iges Spritzpulver

Test

Etwa 4 cm hohe, in Wasser angezogene Erbsenkeimlinge werden vor dem Versuchsbeginn je mit ca. 200 OP-resistenten Individuen der Spezies Myzus persicae besiedelt. Die so behandelten Pflanzen werden 24 Stunden später mit einer wässrigen Suspension enthaltend je 400, 200, 100 oder 50 ppm der zu prüfenden Verbindungen oder deren Kombination bis zur Tropfnässe besprüht. Man verwendet pro Konzentration zwei Pflanzen. Eine Auswertung der erzielten Abtötungsrate erfolgt 48 Stunden nach Applikation. Der Versuch wird bei 20 - 22°C und 60 % relativer Luftfeuchtigkeit durchgeführt.

Testergebnis

% Abtötung von Myzus persicae nach 48 Stunden

| Konzentration | 400 | 200 | 100 | 50 ppm |
|---|---|---|---|---|
| Verbindung A | 0 | 0 | 0 | 0 |
| Verbindung B | 65 | 0 | 0 | 0 |
| Verbindung A+B* | 100 | 90 | 58 | 38 |

* (1:1; d.h. je 200/100/50/25 ppm von beiden Komponenten in der Spritzlösung)

3.8.2.

A. Verbindungen der Formel I gemäss Herstellungsbeispiel 1:

1.1

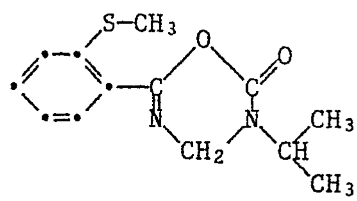

1.11

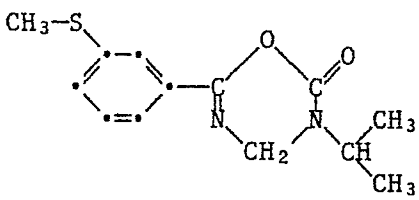

1.13

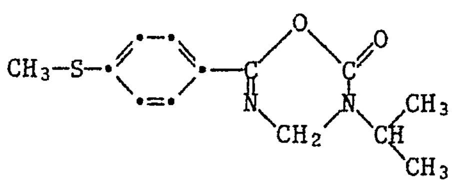

B. Phosphoramidon der Formel

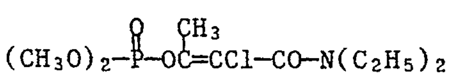

Mit Myzus persicae (OP-resistent) besetzte Erbsenkeimlinge werden in wässrige Mischungen der Testsubstanzen und Phosphamidon (im Handel erhältlich unter der Bezeichnung Dimecron) der angegebenen Konzentrationen [ppm] eingetaucht. Nach 48 Stunden wird die Mortalität der Blattläuse in % bestimmt:

Testergebnis

| Mischung A:B / Verb. n. Beisp. | % Mortalität | | | | | | |
|---|---|---|---|---|---|---|---|
| | 60:0 | 50:10 | 40:10 | 30:10 | 20:10 | 10:10 | 0:60 |
| 1.1 | 0 | 80 | 80 | 40 | 40 | 0 | - |
| 1.11 | 30 | 90 | 90 | 70 | 40 | 20 | - |
| 1.13 | 0 | 90 | 90 | 80 | 60 | 0 | - |
| B | - | - | - | - | - | - | 0 |

**Patentansprüche**

1. Oxadiazinone der Formel I

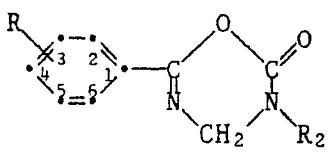

(I),

worin
R $S(O)_n-R_1$;
$R_1$ $C_1$-$C_6$-Alkyl;
$R_2$ $C_1$-$C_6$-Alkyl oder $C_3$-$C_6$-Cycloalkyl und
n eine Zahl 0 oder 1 bedeuten,
mit der Massgabe, dass $R_2$ eine von Methyl abweichende Bedeutung hat, wenn R für 4-$SOCH_3$ steht.

2. Verbindungen der Formel I nach Anspruch 1, dadurch gekennzeichnet, dass
$R_1$ $C_1$-$C_4$-Alkyl und
$R_2$ $C_1$-$C_4$-Alkyl, Cyclopropyl oder Cyclohexyl
bedeuten.

3. Verbindungen der Formel I nach Anspruch 1 oder 2, dadurch gekennzeichnet, dass n = 0 ist.

4. Verbindungen der Formel I nach Anspruch 2 oder 3, dadurch gekennzeichnet, dass
$R_1$ Methyl und
$R_2$ Isopropyl
bedeuten.

5. Verbindung gemäss Anspruch 4 der Formel

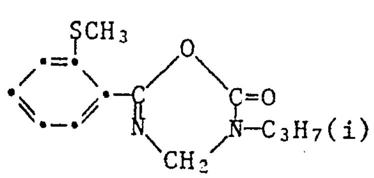

.

6. Verbindung gemäss Anspruch 4 der Formel

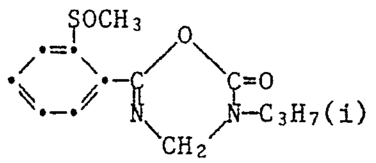

.

7. Verbindung gemäss Anspruch 3 der Formel

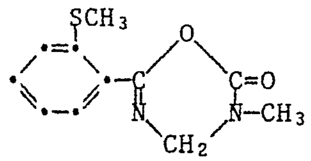

.

8. Verbindung gemäss Anspruch 3 der Formel

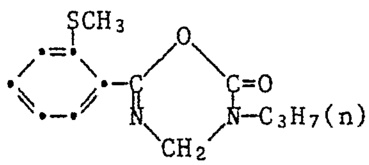

.

9. Verbindung gemäss Anspruch 3 der Formel

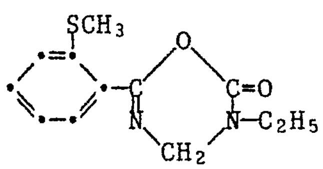

.

10. Verbindung gemäss Anspruch 3 der Formel

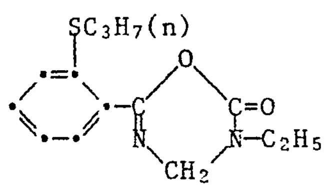

.

11. Verbindung gemäss Anspruch 3 der Formel

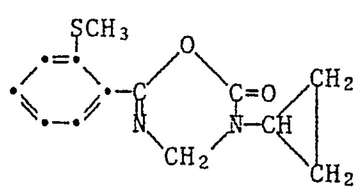

.

12. Verbindung gemäss Anspruch 3 der Formel

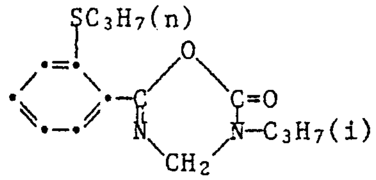 .

13. Verbindung gemäss Anspruch 3 der Formel

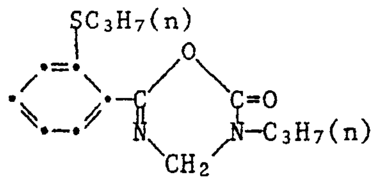 .

14. Verbindung gemäss Anspruch 4 der Formel

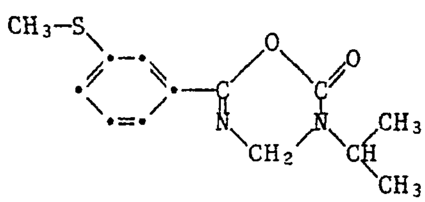 .

15. Verbindung gemäss Anspruch 4 der Formel

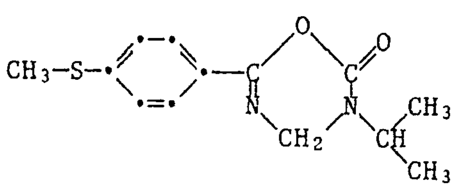 .

16. Verfahren zur Herstellung von Verbindungen gemäss einem der Ansprüche 1 bis 15, dadurch gekennzeichnet, dass man

a) eine Verbindung der Formel II

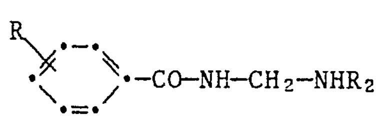 (II),

worin R, $R_1$, $R_2$ und n die in den Ansprüchen 1 bis 4 angegebenen Bedeutungen haben, mit Phosgen

cyclisiert oder

b) indem man eine Verbindung der Formel II durch Umsetzung einer Verbindung der Formel III

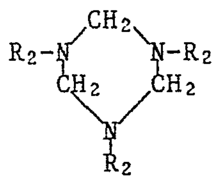

(III)

mit einem Benzamid der Formel IV

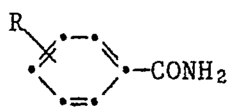

(IV),

in situ herstellt und dann mit Phosgen direkt cyclisiert, wobei in den Formeln II bis IV, R, $R_1$, $R_2$ und n die in den Ansprüchen 1 bis 4 angegebenen Bedeutungen haben.

17. Insektizide und akarizide Mittel enthaltend als aktive Komponente mindestens eine Verbindung der Formel I,

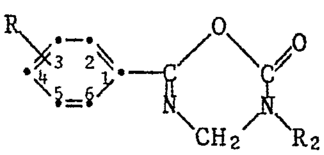

(I),

worin
R $S(O)_n$-$R_1$;
$R_1$ $C_1$-$C_6$-Alkyl;
$R_2$ $C_1$-$C_6$-Alkyl oder $C_3$-$C_6$-Cycloalkyl und
n eine Zahl 0, 1 oder 2 bedeuten, zusammen mit geeigneten Hilfs-und/oder Trägerstoffen

18. Mittel gemäss Anspruch 17, enthaltend als aktive Komponente eine Verbindung der Formel I,

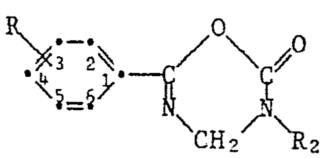

(I),

worin
R $S(O)_n$-$R_1$;
$R_1$ $C_1$-$C_6$-Alkyl;
$R_2$ $C_1$-$C_6$-Alkyl oder $C_3$-$C_6$-Cycloalkyl und
n eine Zahl 0 oder 1 bedeuten
mit der Massgabe, dass $R_2$ eine von Methyl abweichende Bedeutung hat, wenn R für 4-$SOCH_3$ steht.

19. Mittel gemäss einem der Ansprüche 17 oder 18, dadurch gekennzeichnet, dass es als aktive Komponente neben einer Verbindung der Formel I einen weiteren insektiziden und/oder akariziden Wirkstoff enthält.

20. Mittel nach Anspruch 19 enthaltend Phosphamidon und eine Verbindung der Formel I als Wirkstoffe.

21. Verwendung von Verbindungen der Formel I
worin
R $S(O)_n$-$R_1$;
$R_1$ $C_1$-$C_6$-Alkyl;
$R_2$ $C_1$-$C_6$-Alkyl oder $C_3$-$C_6$-Cycloalkyl und
n eine Zahl 0, 1 oder 2 bedeuten,
zur Bekämpfung von Insekten und Vertretern der Ordnung Acarina an Tieren und Pflanzen sowie im Boden.

22. Verwendung gemäss Anspruch 21 zur Bekämpfung von pflanzenschädigenden Insekten.

23. Verwendung einer Kombination einer Verbindung der Formel I
worin
R $S(O)_n$-$R_1$;
$R_1$ $C_1$-$C_6$-Alkyl;
$R_2$ $C_1$-$C_6$-Alkyl oder $C_3$-$C_6$-Cycloalkyl und
n eine Zahl 0, 1 oder 2 bedeuten,
und eines weiteren insektiziden und/oder akariziden Wirkstoffes zur Bekämpfung von Insekten und Vertretern der Ordnung Acarina an Tieren und Pflanzen sowie im Boden.

24. Verfahren zur Bekämpfung von Insekten und Vertretern der Ordnung Acarina an Tieren und Pflanzen sowie im Boden, dadurch gekennzeichnet, dass man die Schädlinge an ihrem Aufenthaltsort mit einer Verbindung der Formel I
worin
R $S(O)_n$-$R_1$;
$R_1$ $C_1$-$C_6$-Alkyl;
$R_2$ $C_1$-$C_6$-Alkyl oder $C_3$-$C_6$-Cycloalkyl und
n eine Zahl 0,1 oder 2 bedeuten,
oder mit einer Kombination einer Verbindung der Formel I mit einem andern insektiziden und/oder akariziden Wirkstoff behandelt.

Europäisches Patentamt

# EUROPÄISCHER RECHERCHENBERICHT

Nummer der Anmeldung

EP 86 81 0574

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | DE-A-2 459 413 (CIBA-GEIGY) * Insgesamt, insbesondere Seite 15, Beispiel 63 * --- | 1-24 | C 07 D 273/04<br>A 01 N 43/88 |
| A | FR-A-2 358 401 (ICI) * Insgesamt * ----- | 17-24 | |
| | | | RECHERCHIERTE SACHGEBIETE (Int. Cl.4) |
| | | | C 07 D 273/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 17-03-1987 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82